(19) **Europäisches Patentamt / European Patent Office / Office européen des brevets**

(11) **EP 3 991 797 A1**

(12) **EUROPEAN PATENT APPLICATION**
published in accordance with Art. 153(4) EPC

(43) Date of publication:
04.05.2022 Bulletin 2022/18

(21) Application number: 20865336.0

(22) Date of filing: 27.08.2020

(51) International Patent Classification (IPC):
$A61P\ 43/00^{(2006.01)}$    $A61P\ 17/00^{(2006.01)}$
$C12N\ 5/071^{(2010.01)}$    $C12N\ 1/20^{(2006.01)}$
$C12Q\ 1/02^{(2006.01)}$    $A61K\ 35/12^{(2015.01)}$
$A61K\ 35/33^{(2015.01)}$    $A61K\ 35/35^{(2015.01)}$
$A61K\ 35/36^{(2015.01)}$    $A61K\ 35/44^{(2015.01)}$
$A61L\ 27/24^{(2006.01)}$    $A61L\ 27/36^{(2006.01)}$
$A61L\ 27/38^{(2006.01)}$    $A61L\ 27/40^{(2006.01)}$
$A61L\ 27/60^{(2006.01)}$

(52) Cooperative Patent Classification (CPC):
A61K 35/12; A61K 35/33; A61K 35/35;
A61K 35/36; A61K 35/44; A61L 27/24;
A61L 27/36; A61L 27/38; A61L 27/40; A61L 27/60;
A61P 17/00; A61P 43/00; C12N 1/20; C12Q 1/02

(86) International application number:
PCT/JP2020/032446

(87) International publication number:
WO 2021/054079 (25.03.2021 Gazette 2021/12)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 17.09.2019 JP 2019168577

(71) Applicants:
• TOPPAN INC.
Tokyo 110-0016 (JP)
• Osaka University
Suita-shi, Osaka 565-0871 (JP)

(72) Inventors:
• KITANO Shiro
Tokyo 110-0016 (JP)
• IRIE Shinji
Tokyo 110-0016 (JP)
• MATSUSAKI Michiya
Suita-shi, Osaka 565-0871 (JP)
• SASAKI Naoko
Suita-shi, Osaka 565-0871 (JP)
• AMANO Atsuo
Suita-shi, Osaka 565-0871 (JP)
• TAKEUCHI Hiroki
Suita-shi, Osaka 565-0871 (JP)

(74) Representative: TBK
Bavariaring 4-6
80336 München (DE)

(54) **CELL STRUCTURE AND METHOD FOR PRODUCING SAME**

(57) Provided is a cell structure including: a connective tissue structure; and an epithelial structure placed on the connective tissue structure, in which the connective tissue structure contains a fragmented extracellular matrix component and first cells including mesenchymal cells, at least a part of the fragmented extracellular matrix component is placed between the first cells, and the epithelial structure contains epithelial cells.

EP 3 991 797 A1

*Fig.1*

(A)

1mm

(B)

b

a

200 μm

## Description

### Technical Field

The present invention relates to a cell structure and a method for producing the same. The present invention also relates to a bacteria-containing tissue model and a method for producing the same, and a method for evaluating the behavior of a cell structure and/or bacteria.

### Background Art

As a method for artificially producing a structure imitating a biological tissue, a method for producing a three-dimensional tissue construct, the method including three-dimensionally arranging cells coated with a collagen-containing coating film to form a three-dimensional tissue construct (Patent Literature 1) and a method for producing a three-dimensional cell tissue, the method including mixing cells with a cationic substance and an extracellular matrix component to obtain a mixture, collecting cells from the obtained mixture, and forming a cell aggregate on a substrate (Patent Literature 2) are known, for example. In addition, the present inventors have proposed a method for producing a three-dimensional tissue construct with a large diameter, a thickness of greater than or equal to 1 mm, and a relatively small number of cells by bringing cells into contact with fragmented exogenous collagen (Patent Literature 3). It is expected that such three-dimensional tissue constructs will be able to be used as substitutes for laboratory animals, implant materials, or the like.

### Citation List

### Patent Literature

[Patent Literature 1] PCT International Publication No. WO2015/072164
[Patent Literature 2] PCT International Publication No. WO2017/146124
[Patent Literature 3] PCT International Publication No. WO2018/143286

## Summary of Invention

### Technical Problem

An object of the present invention is to provide a cell structure closer to a biological tissue, and a method for producing the same.

### Solution to Problem

That is, the present invention relates to, for example, each of the following inventions.

[1] A cell structure including: a connective tissue structure; and an epithelial structure placed on the connective tissue structure, in which the connective tissue structure contains a fragmented extracellular matrix component and first cells including mesenchymal cells, at least a part of the fragmented extracellular matrix component is placed between the first cells, and the epithelial structure contains epithelial cells.
[2] The cell structure according to [1], in which the mesenchymal cells include fibroblasts.
[3] The cell structure according to [1] or [2], in which the fragmented extracellular matrix component contains a fragmented collagen component.
[4] The cell structure according to any one of [1] to [3], in which the first cells include vascular endothelial cells, and the connective tissue structure has a vascular network between the first cells.
[5] The cell structure according to [4], in which the connective tissue structure has a vascular network between the mesenchymal cells.
[6] A bacteria-containing tissue model including: the cell structure according to any one of [1] to [5]; and bacteria arranged in the cell structure or arranged so as to come into contact with the epithelial structure of the cell structure.
[7] The bacteria-containing tissue model according to [6], in which the bacteria are periodontal disease bacteria.
[8] A method for producing a cell structure, the method including: a first step of bringing a fragmented extracellular matrix component into contact with first cells including mesenchymal cells and culturing the first cells to form a connective tissue structure; and a second step of bringing second cells including epithelial cells into contact with the connective tissue structure and culturing the connective tissue structure and the second cells to form an epithelial structure on the connective tissue structure and obtain a cell structure.

[9] The method for producing a cell structure according to [8], in which the first cells include vascular endothelial cells.

[10] The method for producing a cell structure according to [8] or [9], in which the amount of the fragmented extracellular matrix component when the fragmented extracellular matrix component is brought into contact with the first cells is 0.1 to 100 mg per $1.0 \times 10^6$ cells.

[11] A method for producing a bacteria-containing tissue model, the method including: a step of bringing bacteria into contact with the epithelial structure of the cell structure obtained through the production method according to any one of [8] to [10].

[12] The method for producing a bacteria-containing tissue model according to [11], the method further including: a step of culturing the cell structure and the bacteria after the step of bringing the bacteria into contact with the epithelial structure to move the bacteria to the inside of the cell structure.

[13] A method for evaluating behavior of a cell structure and/or bacteria, the method including: a step of evaluating behavior of the cell structure and/or the bacteria after bringing the bacteria into contact with the epithelial structure of the cell structure according to any one of [1] to [5].

**Advantageous Effects of Invention**

According to the present invention, it is possible to provide a cell structure closer to a biological tissue, and a method for producing the same.

**Brief Description of Drawings**

Fig. 1 shows photographs illustrating microscopic observation results of a cell structure stained with hematoxylin and eosin (HE).

Fig. 2 is a photograph illustrating formation of a vascular network in a connective tissue in a cell structure.

Fig. 3 shows photographs illustrating confirmation results (image analysis) of engraftment and infiltration of periodontal disease bacteria.

Fig. 4 shows photographs illustrating confirmation results of engraftment and infiltration of *P. gingivalis* with respect to a cell structure having a vascular network.

Fig. 5 is a graph illustrating quantitative evaluation results of *P. gingivalis* colocalized with vascular networks.

Fig. 6 is a graph illustrating quantitative evaluation results of the number of *P. gingivalis* infiltrated into each gingival model tissue.

Fig. 7 is a graph illustrating evaluation results of the amount of IL-6 secreted after *P. gingivalis* infection.

Fig. 8 is a view illustrating a method for evaluating behavior of *P. gingivalis* permeating an epithelial structure.

Fig. 9 is a graph illustrating evaluation results of behavior of *P. gingivalis* permeating the epithelial structure.

**Description of Embodiments**

Hereinafter, embodiments of the present invention will be described in detail. However, the present invention is not limited to the following embodiments.

<Cell Structure>

A cell structure according to the present embodiment includes: a connective tissue structure; and an epithelial structure placed on the connective tissue structure. In the cell structure according to the present embodiment, the connective tissue structure contains a fragmented extracellular matrix component and first cells including mesenchymal cells, and at least a part of the fragmented extracellular matrix component is placed between the first cells. In the cell structure according to the present embodiment, the epithelial structure contains epithelial cells.

The cell structure can be used, for example, as a biological tissue model. Specific examples of a biological tissue model include a gingival model, an oral mucosa model, a skin model, and a corneal model.

In the present specification, the "cell structure" means a cell aggregate in which cells are three-dimensionally arranged through extracellular matrix components and which is artificially produced through cell culture. The shape of the cell structure is not particularly limited, and examples thereof include a sheet shape, a spherical shape, an ellipsoidal shape, and a rectangular parallelepiped shape. Here, a biological tissue includes sweat glands, lymph vessels, sebaceous glands, and the like and has a more complicated structure than the cell structure. For this reason, it is possible to easily distinguish the cell structure from a biological tissue.

(Cells)

"Cells" in the present specification are not particularly limited, but cells may be derived from mammals such as humans, monkeys, dogs, cats, rabbits, pigs, cattle, mice, and rats, for example. The origin of the cells is also not particularly limited, but the cells may be somatic cells derived from the bones, the muscles, the internal organs, nerves, the brain, the bones, the skin, blood, or the like, or may be reproductive cells. Furthermore, the cells may be stem cells or may be cultured cells such as primary culture cells, subcultured cells, and cell line cells.

"Stem cells" in the present specification mean cells having a self-replication ability and a pluripotency. The stem cells include pluripotent stem cells having an ability to differentiate into an arbitrary cell tumor and tissue stem cells (also called somatic stem cells) having an ability to differentiate into a specific cell tumor. Examples of pluripotent stem cells include embryonic stem cells (ES cells), somatic cell-derived ES cells (ntES cells), and induced pluripotent stem cells (iPS cells). Examples of tissue stem cells include mesenchymal stem cells (for example, adipose stem cells and bone marrow-derived stem cells), hematopoietic stem cells, and neural stem cells. Examples of adipose stem cells include human adipose stem cells (ADSC).

A remaining proportion of a cell structure subjected to trypsin treatment at a trypsin concentration of 0.25%, a temperature of 37°C, a pH of 7.4, and a reaction time of 15 minutes may be greater than or equal to 70%, greater than or equal to 80%, or greater than or equal to 90%. Such a cell structure is stable because it is unlikely to be decomposed by an enzyme during or after culturing. The above-described remaining proportion can be calculated from the mass of a cell structure before and after trypsin treatment, for example.

The remaining proportion of a cell structure subjected to collagenase treatment at a collagenase concentration of 0.25%, a temperature of 37°C, a pH of 7.4, and a reaction time of 15 minutes may be greater than or equal to 70%, greater than or equal to 80%, or greater than or equal to 90%. Such a cell structure is stable because it is unlikely to be decomposed by an enzyme during or after culturing.

The thickness of the above-described cell structure is preferably greater than or equal to 10 $\mu$m, more preferably greater than or equal to 100 $\mu$m, and still more preferably greater than or equal to 1,000 $\mu$m. Such a cell structure is a structure closer to a biological tissue, and is suitable as a substitute for a laboratory animal and an implant material. The upper limit of the thickness of a cell structure is not particularly limited, but may be, for example, less than or equal to 10 mm, less than or equal to 3 mm, less than or equal to 2 mm, less than or equal to 1.5 mm, and less than or equal to 1 mm.

Here, the "thickness of a cell structure" means the distance between both ends in a direction perpendicular to the main surface in a case where the cell structure has a sheet shape or a rectangular parallelepiped shape. In a case where the above-described main surface is uneven, the thickness means the distance therebetween at the thinnest portion of the above-described main surface.

In addition, in a case where a cell structure has a spherical shape, the thickness thereof means the diameter thereof. In addition, in a case where a cell structure has an ellipsoidal shape, the thickness thereof means the minor axis thereof. In a case where a cell structure has a substantially spherical shape or a substantially ellipsoidal shape and its surface is uneven, the thickness thereof means the shortest distance between two points where a straight line passing through the center of gravity of the cell structure and the above-described surface intersect.

[Connective Tissue Structure]

A connective tissue structure contains a fragmented extracellular matrix component and first cells. At least a part of the fragmented extracellular matrix component is placed between the first cells. By using a fragmented extracellular matrix component in a cell structure, a connective tissue structure containing higher-density extracellular matrix molecules can be formed, and a thicker connective tissue structure is likely formed.

(Fragmented Extracellular Matrix Components)

The "extracellular matrix components" in the present specification are extracellular matrix molecule aggregates formed by a plurality of extracellular matrix molecules. The extracellular matrix means a substance existing outside cells in an organism. An arbitrary substance can be used as the extracellular matrix as long as this does not adversely affect growth of cells and formation of cell aggregates. Specific examples thereof include collagen, elastin, proteoglycans, fibronectin, hyaluronic acid, laminin, vitronectin, tenascin, entactin, and fibrillin, but the present invention is not limited thereto. These extracellular matrix components may be used alone or in combination. Extracellular matrix components may, for example, contain collagen components or consist of collagen components. The extracellular matrix components in the present embodiment are preferably substances existing outside animal cells, that is, animal extracellular matrix components. Extracellular matrix molecules may be modified extracellular matrix molecules or variants of extracellular matrix molecules or may be polypeptides such as chemically synthesized peptides as long as these do not adversely affect the growth of cells and the formation of cell aggregates.

The "fragmentation" means that an aggregate of extracellular matrix components is made smaller in size. Fragmented extracellular matrix components may include defibrated extracellular matrix components. Defibrated extracellular matrix components are components obtained by defibrating the above-described extracellular matrix components through application of physical force. Defibration is performed under the conditions of, for example, not cleaving the bond within extracellular matrix molecules.

The shape of a fragmented extracellular matrix component may be arbitrary. Specific examples thereof include a fibrous shape, a spindle shape, an amorphous shape, a spherical shape, a granular shape, a powdery shape, a flat shape, and a sheet shape, but the present invention is not limited thereto. It is preferable that the shape thereof be the same as or similar to that of an extracellular matrix component in an organism. For example, in a case where an extracellular matrix component is collagen, the shape of a fragmented extracellular matrix component is preferably fibrous.

The method for fragmenting an extracellular matrix component such as a collagen component is not particularly limited but may be performed through application of physical force. The method for fragmenting an extracellular matrix component may be, for example, a method for finely crushing a bulk extracellular matrix component. The extracellular matrix component may be fragmented as it is. That is, the extracellular matrix component may be fragmented in a solid phase. In addition, the extracellular matrix component may be fragmented in an aqueous medium. The fragmentation of an extracellular matrix component through physical force can be performed, for example, using an ultrasonic homogenizer, a stirring homogenizer, and a high-pressure homogenizer. In a case of using a stirring homogenizer, extracellular matrix components may be homogenized as they are or may be homogenized in an aqueous medium such as physiological saline. In addition, millimeter-sized and nanometer-sized fragmented extracellular matrix components can also be obtained by adjusting the time, the number of times of homogenizing or the like.

The size of pieces of fragmented extracellular matrix components can be obtained, for example, by analyzing each piece of fragmented extracellular matrix component using an electron microscope. The size of a piece of fibrous fragmented extracellular matrix component may be a length in a longitudinal direction, and the size of a piece of fragmented extracellular matrix component with a shape other than a fibrous shape may be a maximum length of a straight line connecting one end to the other end of the piece of fragmented extracellular matrix component.

The average size of pieces of fragmented extracellular matrix components may be 100 nm to 400 $\mu$m and 100 nm to 200 $\mu$m. In one embodiment, the average size of pieces of fragmented extracellular matrix components may be 5 $\mu$m to 400 $\mu$m, 10 $\mu$m to 400 $\mu$m, or 100 $\mu$m to 400 $\mu$m from the viewpoint of facilitating formation of thick tissue. In another embodiment, the average size of pieces of fragmented extracellular matrix components may be less than or equal to 100 $\mu$m, less than or equal to 50 $\mu$m, less than or equal to 30 $\mu$m, less than or equal to 15 $\mu$m, less than or equal to 10 $\mu$m, or less than or equal to 1 $\mu$m, and greater than or equal to 100 nm. Of all the pieces of fragmented extracellular matrix components, the average size of most of the pieces of fragmented extracellular matrix components is preferably within the above-described numerical ranges. Specifically, of all the pieces of fragmented extracellular matrix components, the average size of 50% or more of the pieces of fragmented extracellular matrix components is preferably within the above-described numerical ranges, and the average size of 95% or more of the pieces of fragmented extracellular matrix components is more preferably within the above-described numerical ranges. The fragmented extracellular matrix components are preferably fragmented collagen components having an average size within the above-described ranges.

The average length of pieces of fragmented extracellular matrix components may be 100 nm to 400 $\mu$m and 100 nm to 200 $\mu$m. In one embodiment, the average length of pieces of fragmented extracellular matrix components may be 5 $\mu$m to 400 $\mu$m, 10 $\mu$m to 400 $\mu$m, or 100 $\mu$m to 400 $\mu$m from the viewpoint of facilitating formation of thick tissue. In another embodiment, the average length of pieces of fragmented extracellular matrix components may be less than or equal to 100 $\mu$m, less than or equal to 50 $\mu$m, less than or equal to 30 $\mu$m, less than or equal to 15 $\mu$m, less than or equal to 10 $\mu$m, or less than or equal to 1 $\mu$m, and greater than or equal to 100 nm. Of all the fragmented extracellular matrix components, the average length of most of the pieces of fragmented extracellular matrix components is preferably within the above-described numerical ranges. Specifically, of all the fragmented extracellular matrix components, the average length of 50% or more of the pieces of fragmented extracellular matrix components is preferably within the above-described numerical ranges, and the average length of 95% or more of the pieces of fragmented extracellular matrix components is more preferably within the above-described numerical ranges. Fragmented extracellular matrix components are preferably fragmented collagen components having an average length within the above-described ranges.

The average diameter of pieces of fragmented extracellular matrix components may be 50 nm to 30 $\mu$m, 4 $\mu$m to 30 $\mu$m, or 5 $\mu$m to 30 $\mu$m. Fragmented extracellular matrix components are preferably fragmented collagen components having an average diameter within the above-described ranges.

The average length and the average diameter of pieces of fragmented extracellular matrix components can be obtained by measuring each fragmented extracellular matrix component using an optical microscope or the like and performing image analysis. In the present specification, the "average length" means an average value of the lengths of the measured samples in the longitudinal direction and the "average diameter" means an average value of the lengths of the measured samples in the direction orthogonal to the longitudinal direction.

In a case where an extracellular matrix component is a collagen component, a fragmented extracellular matrix component is also referred to as a "fragmented collagen component." A "fragmented collagen component" is one obtained by fragmenting a collagen component such as a fibrous collagen component and means that it maintains a triple helix structure. The average length of pieces of fragmented collagen components is preferably 100 nm to 200 $\mu$m, more preferably 22 $\mu$m to 200 $\mu$m, and still more preferably 100 $\mu$m to 200 $\mu$m. The average diameter of pieces of fragmented collagen components is preferably 50 nm to 30 $\mu$m, more preferably 4 $\mu$m to 30 $\mu$m, and still more preferably 20 $\mu$m to 30 $\mu$m.

At least some fragmented extracellular matrix components may be cross-linked between or within molecules. The extracellular matrix components may be cross-linked within or between extracellular matrix molecules constituting the extracellular matrix components.

Examples of cross-linking methods include a physical cross-linking method through application of heat, ultraviolet rays, radiation, or the like and a chemical cross-linking method using a cross-linking agent or through an enzymatic reaction or the like, but the methods thereof are not particularly limited. Physical cross-linking is preferable from the viewpoint of not hindering cell growth. Cross-linking (physical cross-linking and chemical cross-linking) may be performed through a covalent bond.

In a case where an extracellular matrix component contains a collagen component, cross-linking may be formed between collagen molecules (triple helix structure) or may be formed between collagen fibrils formed by collagen molecules. The cross-linking may be cross-linking by heat (thermal cross-linking). Thermal cross-linking can be carried out, for example, by performing heat treatment under reduced pressure using a vacuum pump. In a case of thermal cross-linking collagen components, extracellular matrix components may be cross-linked such that amino groups of collagen molecules form peptide bonds (-NH-CO-) with carboxyl groups of the same or other collagen molecules.

Extracellular matrix components can also be cross-linked using a cross-linking agent. The cross-linking agent may be one capable of cross-linking carboxyl groups with amino groups or one capable of cross-linking amino groups. Aldehyde-based, carbodiimide-based, epoxide-based, and imidazole-based cross-linking agents are preferable as cross-linking agents from the viewpoints of economical efficiency, safety, and operability, and specific examples thereof include water-soluble carbodiimides such as glutaraldehyde, 1-ethyl-3-(3-dimethylaminopropyl)carbodiimide hydrochloride and 1-cyclohexyl-3-(2-morpholinyl-4-ethyl)carbodiimide sulfonate.

The quantitative determination of the degree of cross-linking can be appropriately selected depending on the types of extracellular matrix components, means for cross-linking extracellular matrix components, and the like. The degree of cross-linking may be greater than or equal to 1%, greater than or equal to 2%, greater than or equal to 4%, greater than or equal to 8%, or greater than or equal to 12%, and less than or equal to 30%, less than or equal to 20%, or less than or equal to 15%. When the degree of cross-linking is within the above-described ranges, extracellular matrix molecules can be suitably dispersed and redispersibility after dry storage is favorable.

In a case where an amino group in an extracellular matrix component is used for cross-linking, the degree of cross-linking can be quantitatively determined based on a TNBS (2,4,6-trinitrobenzene sulfonic acid) method disclosed in Acta Biomaterialia, 2015, Vol. 25, pp. 131-142 or the like. The degree of cross-linking obtained through the TNBS method may be within the above-described ranges. The degree of cross-linking obtained through the TNBS method is a proportion of amino groups used for cross-linking among amino groups in the extracellular matrix. In a case where an extracellular matrix component contains a collagen component, the degree of cross-linking measured through the TNBS method is preferably within the above-described range.

The degree of cross-linking may be calculated by quantitatively determining carboxyl groups. For example, in a case of extracellular matrix components which are insoluble in water, the degree of cross-linking may be quantitatively determined through a toluidine blue O (TBO) method. The degree of cross-linking obtained through the TBO method may be within the above-described ranges.

The content of extracellular matrix components in a cell structure based on the dry weight of a connective tissue structure may be 0.01 to 90 mass%, preferably 10 to 90 mass%, 10 to 80 mass%, 10 to 70 mass%, 10 to 60 mass%, 1 to 50 mass%, and 10 to 50 mass%, and more preferably 10 to 30 mass% and 20 to 30 mass%.

Here, the "extracellular matrix component in a cell structure" means an extracellular matrix component constituting a cell structure, and may be derived from an endogenous extracellular matrix component or may be derived from an exogenous extracellular matrix component.

The "endogenous extracellular matrix component" means an extracellular matrix component produced by extracellular matrix-producing cells. Examples of extracellular matrix-producing cells include the above-described mesenchymal cells such as fibroblasts, chondrocytes, and osteoblasts. The endogenous extracellular matrix component may be fibrous or may be non-fibrous.

The "exogenous extracellular matrix component" means an extracellular matrix component supplied from outside. The cell structure according to the present embodiment contains a fragmented extracellular matrix component which is an exogenous extracellular matrix component. An animal species from which an exogenous extracellular matrix component is derived may be the same as or different from that of an endogenous extracellular matrix component. Examples of animal species from which an exogenous extracellular matrix component is derived include humans, pigs, and cattle. In addition, an exogenous extracellular matrix component may be an artificial extracellular matrix component.

In a case where an extracellular matrix component is a collagen component, an exogenous extracellular matrix component is also referred to as an "exogenous collagen component" which means a collagen component supplied from outside and is a collagen molecule aggregate formed of a plurality of collagen molecules. Specific examples thereof include fibrous collagen and non-fibrous collagen. The exogenous collagen component is preferably fibrous collagen. The above-described fibrous collagen means a collagen component that is a main component of collagen fibers, and examples thereof include type I collagen, type II collagen, and type III collagen. Commercially available collagen may be used as the above-described fibrous collagen, and specific examples thereof include porcine skin-derived type I collagen manufactured by NH Foods Ltd. Examples of exogenous non-fibrous collagen include type IV collagen.

Animal species from which an exogenous extracellular matrix component is derived may be different from those from which cells are derived. In addition, in a case where cells include extracellular matrix-producing cells, animal species from which an exogenous extracellular matrix component is derived may be different from those from which extracellular matrix-producing cells are derived. That is, an exogenous extracellular matrix component may be a heterologous extracellular matrix component.

That is, in a case where a cell structure contains an endogenous extracellular matrix component and a fragmented extracellular matrix component, the above-described content of the extracellular matrix components constituting the cell structure means a total amount of the endogenous extracellular matrix component and the fragmented extracellular matrix component. The above-described content of extracellular matrixes can be calculated from the volume of an obtained cell structure and the mass of a decellularized cell structure.

In a case where an extracellular matrix component contained in a cell structure is, for example, a collagen component, examples of methods for quantitatively determining the collagen component in the cell structure include a method for quantitatively determining hydroxyproline below. Hydrochloric acid (HCl) is mixed with a solution in which a cell structure is dissolved, and the mixed solution is incubated at a high temperature for a predetermined time. Then, the temperature is returned to room temperature, and a centrifuged supernatant is diluted to a predetermined concentration to prepare a sample. A hydroxyproline standard solution is treated in the same manner as the sample, and then diluted stepwise to prepare a standard. Each of the sample and the standard is subjected to a predetermined treatment with hydroxyproline assay buffer and a detection reagent, and the absorbance at 570 nm is measured. The amount of collagen components is calculated by comparing the absorbance of the sample with that of the standard. A solution obtained by directly suspending and dissolving a cell structure in high-concentration hydrochloric acid may be centrifuged, and a supernatant may be collected to be used for quantitative determination of a collagen component. In addition, the cell structure to be dissolved may be in a state where it has been collected from a culture liquid, or may be dissolved in a state where a liquid component has been removed by performing a drying treatment after collection. However, in the case where a cell structure in a state where it has been collected from a culture liquid is dissolved to perform quantitative determination of a collagen component, it is expected that the measurement value of the weight of the cell structure will vary due to the influence of medium components absorbed by the cell structure and the remainder of a medium due to a problem of an experimental technique. Therefore, it is preferable to use the weight after drying as a reference from the viewpoint of stably measuring the amount of collagen component making up the construct per weight and unit weight.

More specific examples of the method for quantitatively determining the amount of collagen component include the following method.

(Preparation of Sample)

The total amount of a freeze-dried cell structure is mixed with 6 mol/L HCl and the mixture is incubated in a heat block at 95°C for 20 hours or longer, and then the temperature is returned to room temperature. After centrifugation at 13,000 g for 10 minutes, a supernatant of the sample solution is collected. After the supernatant is appropriately diluted with 6 mol/L HCl so that measurement results to be described below fall within a range of a calibration curve, 200 μL of the supernatant is diluted with 100 μL of ultrapure water to prepare a sample. 35 μL of the sample is used.

(Preparation of Standard)

125 μL of a standard solution (1,200 μg/mL in acetic acid) and 125 μL of 12 mol/L HCl are placed in a screw-cap tube and mixed with each other, and the mixture is incubated in a heat block at 95°C for 20 hours. Then, the temperature is returned to room temperature. After centrifugation at 13,000 g for 10 minutes, a supernatant is diluted with ultrapure water to prepare 300 $\mu$g/mL S1, and the S1 is diluted stepwise with ultrapure water to prepare S2 (200 $\mu$g/mL), S3 (100 $\mu$g/mL), S4 (50 $\mu$g/mL), S5 (25 $\mu$g/mL), S6 (12.5 $\mu$g/mL), and S7 (6.25 $\mu$g/mL). S8 (0 $\mu$g/mL) containing only 90 $\mu$L of 4 mol/L HCl is also prepared.

(Assay)

35 $\mu$L of each of the standard and the samples is placed on a plate (included in the QuickZyme Total Collagen Assay Kit, QuickZyme Biosciences). 75 $\mu$L of assay buffer (included in the above-described kit) is added to the wells. The plate is closed with a seal and incubated at room temperature while being shaken for 20 minutes. The seal is removed, and 75 $\mu$L of a detection reagent (reagent A : reagent B = 30 $\mu$L:45 $\mu$L, included in the above-described kit) is added to the wells. The plate is closed with a seal, and the solutions are mixed with each other through shaking and incubated at 60°C for 60 minutes. The mixture is sufficiently cooled on ice, and the seal is removed to measure the absorbance at 570 nm. The amount of collagen components is calculated by comparing the absorbance of the sample with that of the standard.

Collagen components occupying a cell structure may be defined by an area ratio or a volume ratio thereof. The "definition by an area ratio or a volume ratio thereof" means that the proportion of a region where collagen components occupying the entire cell structure are present is calculated through naked-eye observation or using various kinds of microscopes, image analysis software, and the like after the collagen components in the cell structure are made to be distinguishable from other tissue components through, for example, a well-known staining technique (for example, immunostaining in which an anti-collagen antibody is used or Masson's Trichrome staining). In a case where the collagen component is defined by the area ratio thereof, there is no limitation on which a cross section or surface in a cell structure defines the area ratio. However, in a case where the cell structure is a spherical body or the like, the collagen component may be defined by a cross-sectional view passing through a substantially central portion thereof.

For example, in a case where the collagen components in a cell structure are defined by an area ratio, the proportion of the area thereof is preferably 0.01% to 99%, 1% to 99%, 5% to 90%, 7% to 90%, or 20% to 90%, and more preferably 50% to 90% based on the total area of the above-described cell structure. The "collagen components in a cell structure" are as described above. The proportion of the area of the collagen components constituting a cell structure means a proportion of the total area of an endogenous collagen component and an exogenous collagen component. The proportion of the area of the collagen components can be calculated, for example, as a proportion of an area of collagen components stained blue with respect to the total cross-sectional area passing through a substantially central portion of an obtained cell structure subjected to Masson's Trichrome staining.

(First cells)

First cells are cells constituting a connective tissue structure in the cell structure according to the present embodiment. The first cells include at least mesenchymal cells. Examples of mesenchymal cells include fibroblasts, osteocytes, chondrocytes, osteoblasts, myocytes, tendon cells, adipocytes, dermal papilla cells, connective tissue cells such as dental pulp cells, and cells having an ability to differentiate into the above-described cells. Mesenchymal cells preferably include fibroblasts from the viewpoints that an endogenous extracellular matrix component is secreted and the bonding force of a connective tissue structure becomes stronger.

The content of mesenchymal cells based on the number of whole cells in a connective tissue structure may be, for example, greater than or equal to 5%, greater than or equal to 10%, greater than or equal to 15%, greater than or equal to 20%, greater than or equal to 25%, or greater than or equal to 30%, and less than or equal to 95%, less than or equal to 90%, less than or equal to 80%, or less than or equal to 75%.

When the cell structure of the present embodiment includes a connective tissue structure containing a fragmented extracellular matrix component and mesenchymal cells, high adhesiveness between cells can be exhibited, for example. As a result, the cell structure according to the present embodiment can be a structure closer to a biological tissue.

The first cells may further include cells other than mesenchymal cells. Examples of the other cells include vascular endothelial cells, cancer cells such as colorectal cancer cells (for example, human colorectal cancer cells (HT29)), and liver cancer cells, cardiomyocytes, lymphatic endothelial cells, nerve cells, dendritic cells, liver cells, adhesive cells (for example, immune cells), smooth muscle cells (for example, aortic smooth muscle cells (Aorta-SMC)), islet cells, and keratinocytes (for example, human epidermal keratinocytes). The first cells may include vascular endothelial cells from the viewpoint that a cell structure closer to a biological tissue can be formed.

The "vascular endothelial cells" in the present specification mean flat cells constituting the surface of the lumen of blood vessels. Examples of vascular endothelial cells include human umbilical vein-derived vascular endothelial cells (HUVEC).

The content of vascular endothelial cells based on the number of whole cells in a connective tissue structure may be, for example, greater than or equal to 5%, greater than or equal to 10%, greater than or equal to 15%, greater than or equal to 20%, greater than or equal to 25%, or greater than or equal to 30%, and less than or equal to 95%, less than or equal to 90%, less than or equal to 80%, or less than or equal to 75%.

(Vascular Network Between Cells)

In a case where first cells include vascular endothelial cells, a connective tissue structure can have a vascular network between cells. The connective tissue structure preferably has a vascular network between mesenchymal cells. "Having a vascular network between cells" means having a structure extending between cells so that branched blood vessels surround the cells, similarly to a biological tissue.

Since a vascular network can be formed between cells like a biological tissue in the cell structure according to the present embodiment, it is expected that even a thick cell structure can be maintained for a long period of time. In addition, it is also expected that the cell structure capable of having a vascular network will be likely to be engrafted when transplanted into a mammal or the like.

Whether or not a vascular network similar to that of a biological tissue is formed can be determined, for example, based on diversity of the number of blood vessel branches in a biological tissue, the length of blood vessels between branches, and/or the diameter of blood vessels. In a case where an average value of the number of blood vessel branches in a cell structure to an average value of the number of blood vessel branches in a biological tissue is, for example, 80% to 150%, 85% to 130%, or 90% to 120%, it may be determined that the number of blood vessel branches in the cell structure is similar to that in the biological tissue.

In a case where an average value of the number of blood vessel branches in a connective tissue structure is, for example, 2.5 to 4.5 or 3.0 to 4.2, it may be determined that the number of blood vessel branches in the connective tissue structure is similar to that in a biological tissue. In a case where an average value of the length of blood vessels between branches in a connective tissue structure to an average value of the length of blood vessels between branches in a biological tissue is, for example, 80% to 150%, 85% to 130%, or 90% to 120%, it may be determined that the length of blood vessels between branches in the connective tissue structure is similar to that in the biological tissue. In the biological tissue, both thick and thin blood vessels are observed. Therefore, in a case where both blood vessels with thick diameters (for example, larger than or equal to 10 $\mu$m and smaller than 25 $\mu$m) and blood vessels with thin diameters (for example, larger than 0 $\mu$m and smaller than 10 $\mu$m) are observed similarly to those in a biological tissue, for example, it may be determined that the connective tissue structure has diversity in diameters of blood vessels similarly to that in the biological tissue. In addition, in a case where 60% or more, 70% or more, or 80% or more of all the diameters of blood vessels is distributed in a range of larger than 0 $\mu$m and smaller than 25 $\mu$m, for example, it may be determined that the connective tissue structure has diversity in diameters of blood vessels similarly to that in the biological tissue.

[Epithelial Structure]

The epithelial structure contains second cells. Second cells are cells constituting an epithelial structure in the cell structure according to the present embodiment. The second cells include at least epithelial cells. Examples of epithelial cells include human gingival epithelial cells. Epithelial cells may be, for example, immortalized epithelial cells transformed from human gingival epithelial cells.

The second cells may further include cells other than epithelial cells. Examples of the other cells include vascular endothelial cells, adipocytes, cancer cells such as colorectal cancer cells (for human colorectal cancer cells (HT29), and liver cancer cells, cardiomyocytes, lymphatic endothelial cells, nerve cells, dendritic cells, liver cells, adhesive cells (for example, immune cells), smooth muscle cells (for example, aortic smooth muscle cells (Aorta-SMC)), islet cells, and keratinocytes (for example, human epidermal keratinocytes).

The content of epithelial cells based on the number of whole cells in an epithelial structure may be, for example, greater than or equal to 5%, greater than or equal to 10%, greater than or equal to 15%, greater than or equal to 20%, greater than or equal to 25%, or greater than or equal to 30%, and less than or equal to 95%, less than or equal to 90%, less than or equal to 80%, or less than or equal to 75%.

<Method for Producing Cell Structure>

A method for producing a cell structure according to the present embodiment includes: a first step of bringing a fragmented extracellular matrix component into contact with first cells including mesenchymal cells and culturing the first cells to form a connective tissue structure; and a second step of bringing second cells including epithelial cells into contact with the connective tissue structure and culturing the connective tissue structure and the second cells to form an epithelial structure on the connective tissue structure and obtain a cell structure.

[First Step]

In a first step, a fragmented extracellular matrix component is brought into contact with first cells including mesenchymal cells (first contact step), and the first cells are cultured (first culture step) in this order to form a connective tissue structure.

The connective tissue structure contains the fragmented extracellular matrix component and the first cells. The fragmented extracellular matrix component and the first cells may be as described above.

If the fragmented extracellular matrix components are dispersed in an aqueous medium, these can be made likely to come into contact with cells in the aqueous medium to promote the formation of the connective tissue structure.

(First Contact Step)

A fragmented extracellular matrix component and first cells may be brought into contact with each other in an aqueous medium. Examples of methods for bringing a fragmented extracellular matrix component into contact with first cells include: a method for mixing an aqueous medium containing a fragmented extracellular matrix component with an aqueous medium containing first cells; a method for adding first cells to an aqueous medium containing a fragmented extracellular matrix component; a method for adding an aqueous medium containing a fragmented extracellular matrix component to a culture liquid containing first cells; a method for adding first cells to an aqueous medium containing a fragmented extracellular matrix component; and a method for adding each of a fragmented extracellular matrix component and first cells to a previously prepared aqueous medium, but the present invention is not limited thereto.

An aqueous medium may be, for example, a liquid medium. The medium is not particularly limited, and a suitable medium can be selected depending on the types of cells to be cultured. Examples of media include an Eagle's MEM medium, DMEM, a Modified Eagle medium (MEM), a Minimum Essential medium, RPMI, and a GlutaMax medium. The medium may be a medium to which serum is added, or may be a serum-free medium. The medium may be a mixed medium obtained by mixing two kinds of media with each other.

A fragmented extracellular matrix component may be brought into contact with first cells after a layer of the first cells is formed in an aqueous medium. That is, the first contact step may be performed by forming a layer of first cells in an aqueous medium and then bringing the layer into contact with a fragmented extracellular matrix component. When a layer of first cells is formed before bringing the first cells into contact with an extracellular matrix component, a connective tissue structure having a high cell density in a lower layer portion can be produced.

A fragmented extracellular matrix component can be obtained through the above-described method. A fragmented extracellular matrix component may be obtained by fragmenting an extracellular matrix component in an aqueous medium. That is, the production method according to the present embodiment may include a step (fragmentation step) of fragmenting an extracellular matrix component in an aqueous medium before the first step. The aqueous medium may be the same as that containing the above-described fragmented extracellular matrix component.

The fragmented extracellular matrix component may be those exemplified above, or may contain a fragmented collagen component.

The production method according to the present embodiment may include a step of heating an extracellular matrix component before the fragmentation step to cross-link at least a part of the extracellular matrix component, or may include a step of heating an extracellular matrix component between the fragmentation step and the contact step to cross-link at least a part of the extracellular matrix component.

In the cross-linking step, the temperature (heating temperature) and the time (heating time) when heating an extracellular matrix component can be appropriately determined. The heating temperature may be, for example, higher than or equal to 100°C or lower than or equal to 200°C, or lower than or equal to 220°C. Specifically, the heating temperature may be, for example, 100°C, 110°C, 120°C, 130°C, 140°C, 150°C, 160°C, 170°C, 180°C, 190°C, 200°C, or 220°C. The heating time (time held at the above-described heating temperatures) can be appropriately set depending on the heating temperature. In a case where heating is performed, for example, at 100°C to 200°C, the heating time may be 6 hours to 72 hours and more preferably 24 hours to 48 hours. In the cross-linking step, heating may be performed in the absence of a solvent or may be performed under a reduced pressure condition.

The production method according to the present embodiment may include a drying step of drying a fragmented extracellular matrix component after the fragmentation step.

In the drying step, the fragmented extracellular matrix component is dried. The drying may be performed, for example, through a freeze-drying method. By performing the drying step after the fragmentation step, an aqueous medium is removed from a liquid containing a fragmented extracellular matrix component and the aqueous medium. Removal of an aqueous medium does not mean that there is no water at all attached to a fragmented extracellular matrix component, but means that there is no water to a degree that can be reasonably reached through the above-described usual drying technique.

The content of mesenchymal cells based on the number of whole first cells in the first contact step may be greater than or equal to 5%, greater than or equal to 10%, greater than or equal to 15%, greater than or equal to 20%, greater than or equal to 25%, or greater than or equal to 30%, and less than or equal to 95%, less than or equal to 90%, less than or equal to 80%, or less than or equal to 75%.

In the first contact step, it is preferable that the first cells further include vascular endothelial cells. By using the fragmented extracellular matrix component and the first cells further including vascular endothelial cells, a connective tissue structure having a vascular network between the cells can be formed.

The content of vascular endothelial cells based on the number of whole first cells in the first contact step may be greater than or equal to 5%, greater than or equal to 10%, greater than or equal to 15%, greater than or equal to 20%, greater than or equal to 25%, or greater than or equal to 30%, and less than or equal to 95%, less than or equal to 90%, less than or equal to 80%, or less than or equal to 75%.

The ratio (mesenchymal cells/vascular endothelial cells) of mesenchymal cells to vascular endothelial cells is not particularly limited, but may be, for example, 100/1 to 1/100, 50/1 to 1/50, 20/1 to 1/1, 10/1 to 1/1, 8/1 to 1/1, 7/1 to 1.2/1, 6/1 to 1.5/1, 5/1 to 2/1, or 3/1 to 2/1.

The concentration of extracellular matrix components in the first contact step can be appropriately determined, for example, depending on the shape and thickness of a target connective tissue structure, the shape and thickness of a cell structure, and the size of an incubator. For example, the concentration of extracellular matrix components in an aqueous media in the first contact step may be 0.1 to 90 mass% or may be 1 to 30 mass%.

The amount of fragmented extracellular matrix component per $1.0 \times 10^6$ cells when the fragmented extracellular matrix component is brought into contact with first cells is, for example, 0.1 to 100 mg, 0.5 to 50 mg, 0.8 to 25 mg, 1.0 to 10 mg, 1.0 to 5.0 mg, 1.0 to 2.0 mg, or 1.0 to 1.8 mg, may be greater than or equal to 0.7 mg, greater than or equal to 1.1 mg, greater than or equal to 1.2 mg, greater than or equal to 1.3 mg, or greater than or equal to 1.4 mg, or may be less than or equal to 7.0 mg, less than or equal to 3.0 mg, less than or equal to 2.3 mg, less than or equal to 1.8 mg, less than or equal to 1.7 mg, less than or equal to 1.6 mg, or less than or equal to 1.5 mg.

In the first contact step, the mass ratio (extracellular matrix components/cells) of fragmented extracellular matrix components to first cells is preferably 1/1 to 1,000/1, more preferably 9/1 to 900/1, and still more preferably 10/1 to 500/1.

(First Culture Step)

The method for culturing first cells is not particularly limited, and a suitable culture method can be performed depending on the types of cells to be cultured. For example, the culture temperature may be 20°C to 40°C or may be 30°C to 37°C. The pH of a medium may be 6 to 8 or may be 7.2 to 7.4. The culture time may be 1 day to 2 weeks or 1 week to 2 weeks.

The cell density in a medium in the first culture step can be appropriately determined, for example, depending on the shape and thickness of a target connective tissue structure, and the size of an incubator. For example, the cell density in a medium in the first culture step may be 1 to $10^8$ cells/mL, or may be $10^3$ to $10^7$ cells/mL. In addition, the cell density in a medium in the first culture step may be the same as that in an aqueous medium in the first contact step.

A step of precipitating extracellular matrix components and first cells together in an aqueous medium may be further provided between the first contact step and the first culture step. By performing such a step, the distribution of the extracellular matrix components and the cells in a connective tissue structure becomes more uniform. The specific method is not particularly limited, but examples thereof include a method for centrifuging a culture liquid containing extracellular matrix components and cells.

[Second Step]

In a second contact step, second cells including epithelial cells are brought into contact with a connective tissue structure (second contact step), and the connective tissue structure and the second cells are cultured (second culture step) to form an epithelial structure on the connective tissue structure and obtain a cell structure.

Collagen (for example, type IV collagen) may be brought into contact with the connective tissue structure as necessary before bringing the second cells including epithelial cells into contact with the connective tissue structure. In this case, a cell structure in which an epithelial structure is placed on a connective tissue body is more likely to be formed. For example, collagen can be brought into contact with a connective tissue structure by holding the connective tissue structure in a solution (collagen solution) containing collagen. The connective tissue structure may be held in a collagen solution at 37°C for 20 minutes or longer, for example. The concentration of collagen in a collagen solution may be, for example, 0.01 to 0.10 mg/mL or 0.02 to 0.06 mg/mL.

The connective tissue structure may be brought into contact with second cells in an aqueous medium. Examples of methods for bringing a connective tissue structure into contact with second cells include: a method for mixing an aqueous medium containing a connective tissue structure with an aqueous medium containing second cells; a method for adding second cells to an aqueous medium containing a connective tissue structure; a method for adding an aqueous medium containing a connective tissue structure to a culture liquid containing second cells; a method for adding second cells to an aqueous medium containing a connective tissue structure; and a method for adding each of a connective tissue structure and cells to a previously prepared aqueous medium, but the present invention is not limited thereto.

An aqueous medium may be, for example, a liquid medium. The medium is not particularly limited, and a suitable medium can be selected depending on the types of cells to be cultured. The medium may be the same or different kind of medium used in the first step. Examples of media include an Eagle's MEM medium, DMEM, a Modified Eagle medium (MEM), a Minimum Essential medium, RPMI, and a GlutaMax medium. The medium may be a medium to which serum is added, or may be a serum-free medium. The medium may be a mixed medium obtained by mixing two kinds of media with each other.

The content of epithelial cells in the second contact step based on the number of whole second cells may be greater than or equal to 5%, greater than or equal to 10%, greater than or equal to 15%, greater than or equal to 20%, greater than or equal to 25%, or greater than or equal to 30%, and less than or equal to 95%, less than or equal to 90%, less than or equal to 80%, or less than or equal to 75%.

(Second Culture Step)

The method for culturing a connective tissue structure and second cells is not particularly limited, and a suitable culture method can be performed depending on the types of cells to be cultured. For example, the culture temperature may be 20°C to 40°C or may be 30°C to 37°C. The pH of a medium may be 6 to 8 or may be 7.2 to 7.4. The culture time may be 1 day to 2 weeks or 1 week to 2 weeks.

The medium is not particularly limited, and a suitable medium can be selected depending on the types of cells to be cultured. The medium may be, for example, the above-described media.

The cell density in a medium in the second culture step can be appropriately determined, for example, depending on the shape and thickness of a target cell structure, and the size of an incubator. For example, the cell density in a medium in the second culture step may be 1 to $10^8$ cells/mL, or may be $10^3$ to $10^7$ cells/mL. In addition, the cell density in a medium in the second culture step may be the same as that in an aqueous medium in the second contact step.

The shrinkage rate of the cell structure produced through the production method according to the present embodiment during culture is preferably less than or equal to 20%, more preferably less than or equal to 15%, and still more preferably less than or equal to 10%. The above-described shrinkage rate can be calculated, for example, by the following equation. L1 in the equation indicates a length of the longest portion of the cell structure on day 1 after culture, and L3 indicates a length of a portion corresponding to the cell structure on day 3 after culture.

$$\text{Shrinkage rate (\%)} = \{(L1-L3)/L1\} \times 100$$

In the above-described example, the shrinkage rate is calculated from the cell structure on day 1 after culture and the cell structure on day 3 after culture. However, the shrinkage rate may be calculated from a cell structure at an arbitrary point in time during a culture period including a point in time when culture is finished. For example, the shrinkage rate may be calculated from a cell structure on day 1 after culture and a cell structure on day 2 after culture, may be calculated from a cell structure on day 1 after culture and a cell structure on day 5 after culture, or may be calculated from a cell structure on day 1 after culture and a cell structure on day 8 after culture.

After the second culture step, a step of bringing third cells into contact with the cell structure and a step of culturing the third cells may be further provided. The third cells may be the same or different kinds of cells described above. Accordingly, a cell structure with a three-layer structure can be produced. In addition, a cell structure with a plurality of layers can be produced by further repeatedly including a contact step and a culture step, whereby a complicated tissue closer to a living body can be produced.

A step of precipitating connective tissue structures and second cells together in an aqueous medium may be further provided between the second contact step and the second culture step. By performing such a step, the distribution of the extracellular matrix components and the cells in a cell structure becomes more uniform. The specific method is not particularly limited, but examples thereof include a method for centrifuging a culture liquid containing connective tissue structures and second cells.

<Use of Cell Structure>

The cell structure according to the present embodiment can be used, for example, for transplantation. The cell structure according to the present embodiment can have a vascular network between cells like a biological tissue. Accordingly, it is also expected that the cell structure will be likely to be engrafted when transplanted into a mammal or the like.

Animals to be transplanted are not particularly limited, but may be, for example, mammals, and may be humans or non-human animals such as monkeys, dogs, cats, rabbits, pigs, cattle, mice, and rats.

The transplantation method according to the present embodiment includes transplanting the cell structure according to the present embodiment into an animal. Preparing an animal to be transplanted and/or producing a cell structure through the above-described method may be further provided before transplantation. The transplantation method is not particularly limited, but can be performed through a well-known appropriate surgical method or the like according to a transplantation target. The cell structure according to the present embodiment can be applied, for example, to tissue reconstruction.

The method for producing a non-human model animal according to the present embodiment includes transplanting the cell structure according to the present embodiment into a non-human animal. Preparing a non-human animal to be transplanted and/or producing a cell structure through the above-described method may be further provided before transplantation. The non-human model animal according to the present embodiment can be used for applying data obtained from an animal experiment to humans. In order to produce a non-human model animal, it is preferable to use a non-human animal in which rejection for transplants (grafts) is suppressed, for example, a non-human animal with weakened immunity or immunodeficiency.

The cell structure itself according to the present embodiment can also be applied as substitutes for laboratory animals, transplant materials, and the like, and as specific examples, the cell structure can be applied to tissue reconstruction as described above, screening of pathological in vitro models, pharmaceuticals, cosmetics, or medicinal cosmetics (evaluation of drugs) or assay screening of pharmaceuticals, cosmetics, or medicinal cosmetics.

<Bacteria-Containing Tissue Model>

Since the cell structure according to the present embodiment is closer to a biological tissue, it can be suitably used as a tissue model for evaluating behavior of bacteria in a biological tissue (for example, engraftment and/or infiltration of bacteria) and/or an effect of bacteria on the cell structure (for example, behavior of cells positioned around cells brought into contact with bacteria in the cell structure) by placing bacteria in the cell structure.

As one embodiment of the present invention, there is provided a bacteria-containing tissue model including: the cell structure according to the present embodiment; and bacteria arranged in the cell structure or arranged so as to come into contact with the epithelial structure of the cell structure.

Bacteria are not particularly limited as long as they can attach to tissues such as the gingiva, the oral cavity, the skin, and the cornea to act on the tissues. Specific examples of bacteria may include periodontal disease bacteria and oral bacteria. In a case where bacteria are, for example, periodontal disease bacteria, the bacteria-containing tissue model can also be used as periodontal disease bacteria evaluation model. Examples of periodontal disease bacteria include *Prophyromonas gingivalis, Treponema denticola, Prevotella intermedia, Aggregatibacter actinomycetemcomitans, Actinobacicclus actino-mycetemcomitans,* and *Bacteroides forsythus.*

<Method for Producing Bacteria-Containing Tissue Model>

A method for producing a bacteria-containing tissue model according to the present embodiment includes: a step of bringing bacteria into contact with the epithelial structure of the above-described cell structure.

The method for producing a bacteria-containing tissue model may further include: a step of culturing the cell structure and the bacteria after the step of bringing the bacteria into contact with the epithelial structure to move the bacteria to the inside of the cell structure. In the moving step, bacteria may be moved to the inside of the epithelial structure of the cell structure and/or the inside of the connective tissue structure of the cell structure.

<Method for Evaluating Behavior of Cell Structure and/or Bacteria>

As one embodiment of the present invention, there is provided a method for evaluating behavior of a cell structure and/or bacteria, the method including: a step (evaluation step) of evaluating behavior of the cell structure and/or the bacteria after bringing the bacteria into contact with the epithelial structure of the above-described cell structure. The method may include a step (contact step) of bringing the bacteria into contact with the epithelial structure of the above-described cell structure before the evaluation step.

In the method for evaluating behavior of a cell structure and/or bacteria, the evaluation step and/or the contact step may be performed in the presence of chemical substances (for example, pharmaceuticals) For example, the evaluation step may be performed in an environment in which the cell structure is exposed to pharmaceuticals or the like or in an environment in which pharmaceuticals are administered to the cell structure. In the evaluation step, the effect of bacteria on cells positioned around cells brought into contact with the bacteria may be evaluated. In the evaluation step, engraftment and/or infiltration of bacteria may be evaluated. For example, in the evaluation step, routes through which bacteria infiltrate the cell structure can be evaluated.

The evaluation step can be performed, for example, by comparing a cell structure and/or bacteria immediately after bringing the bacteria into contact with an epithelial structure of the cell structure with the cell structure and/or the bacteria after the elapse of a predetermined time (for example, 1 to 72 hours, 2 to 48 hours, 2 to 24 hours, 3 to 8 hours, or 4 to 6 hours) from the contact with the bacteria. For example, nucleic acids contained in the bacteria can be stained, cross-sectional images of the cell structure brought into contact with the bacteria can be acquired, and the acquired cross-sectional images can be compared with each other to evaluate whether or not the bacteria have moved to the inside of the cell structure. For example, whether the bacteria have moved to the inside of the epithelial structure or a connective tissue structure or to a vascular network of the cell structure can be evaluated through the evaluation method according to the present embodiment.

In the evaluation step, the amount of inflammatory cytokine secreted from a cell structure can be evaluated as behavior of the cell structure, for example. The inflammatory cytokine may be, for example, interleukin-6 (IL-6). For example, methods described in examples below can be used as the method for evaluating the amount of inflammatory cytokine.

[Examples]

Hereinafter, the present invention will be described more specifically with reference to the examples. However, the present invention is not limited to these examples.

<Test Example 1: Production of Defibrated Collagen Components>

Porcine skin-derived type I collagen (manufactured by NH Foods Ltd.) which was a lyophilizate was dispersed in a 10-fold concentration of phosphate-buffered physiological saline ($\times$10 PBS) to prepare a type I collagen dispersion. The type I collagen dispersion was homogenized for 5 minutes using a homogenizer to obtain defibrated collagen (hereinafter, also referred to as "CMFs"). The average length of the obtained CMFs was 22.5 $\mu$m, and the average diameter thereof was about 4.4 $\mu$m.

The length of CMFs in the longitudinal direction and the length thereof in a direction orthogonal to the longitudinal direction were obtained by analyzing each CMF with an electron microscope. The average length and the average diameter indicate average values when 391 samples were measured.

The obtained CMFs were washed with a serum-free medium (DMEM) to obtain a CMF medium dispersion. The obtained CMF medium dispersion could be stored for 1 week at room temperature. CMFs obtained through the same method were used as CMFs used in examples to be described below.

<Test Example 2: Production of Cell Structure>

Cells, reagents, production methods, and the like used for producing a cell structure are as follows.

Immortalized human gingival epithelial cells (Epi4)
Culture medium: Humedia-KG2 (KURABO KK2150S)
Human gingival fibroblasts (HGF)
Medium: DMEM 10% FBS p/s
Human umbilical vein-derived vascular endothelial cells (HUVEC): CC-2517 manufactured by Lonza
Medium: EGM2MV (Lonza CC-3202)
*Gingivalis:* 33277 manufactured by ATCC
Medium: 5% sheep blood agar medium (Becton, Dickinson and Company, 252201)
Protease-deficient strain of *gingivalis:* gene knockdown of ATCC strain
Anti-CD31 antibody (Dako, m0823)
DAPI (Invitrogen 62248)
Alexa Fluor 647 Phalloidin (Invitrogen A22287)
Anti-*Prophyromonas gingivlis* (*P. gingivlis*) antibody

Gingival-derived epithelial cells (Epi4), HGF, and Protease-deficient strain of *P. Gingivalis* (ATCC) (gene knockdown of ATCC strain) which were provided by Osaka University, School of Dentistry were used. Preculture of all kinds of cells was performed according to standard procedures.

(Production of Connective Tissue Structure)

4 mg of porcine skin-derived type I collagen (defibrated by homogenization for 5 minutes) manufactured by NH Foods Ltd., $1.0 \times 10^6$ cells of normal human gingival fibroblasts (HGF), and $5.0 \times 10^5$ cells of HUVEC were suspended in a mixed medium in which D-MEM (manufactured by Wako Pure Chemical Industries, Ltd.) and EGM2MV were mixed at a ratio of 1:1, the obtained suspension was added to a 24-well insert (manufactured by Corning), and the mixed medium was added to the outside of the insert to perform culture overnight. Accordingly, a connective tissue structure was produced. Thereafter, the culture was carried out while exchanging the medium every day.

(Production of Epithelial Structure)

The medium of the insert was aspirated 7 days after the formation of the connective tissue structure, the surface of the connective tissue structure was coated with 0.04 mg/mL collagen type IV (manufactured by Sigma-Aldrich), and immortalized human gingival epithelial cells (Epi4) were adjusted so as to become $1.0 \times 10^6$ cells/300 μL/insert and seeded on the connective tissue structure. 1 mL of a mixed medium in which D-MEM, EGM2MV, and Humedia (manufactured by Kurabo Industries Ltd.) were mixed with each other at a ratio of 1:1:1 was placed outside the insert and incubated at 37°C for 1 hour, and then, 1 mL of the mixed medium was added to the outside of the insert to perform culture overnight. Accordingly, a cell structure including a connective tissue structure and an epithelial structure was produced. This cell structure was used as a cell structure for evaluating engraftment and infiltration to be described below.

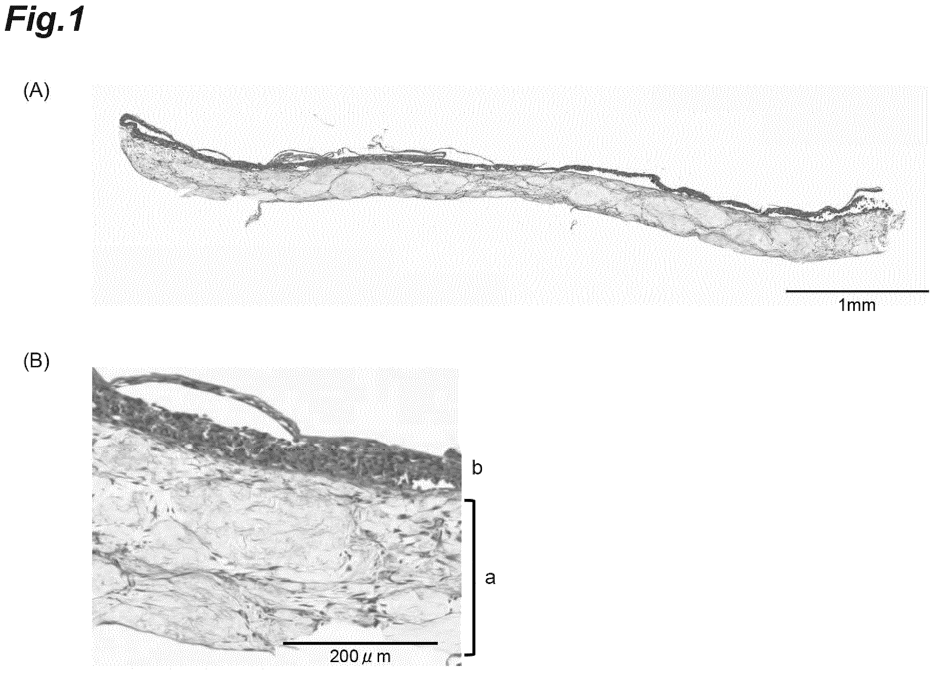

After the Epi4 was seeded and cultured for 1 day, the cell structure was fixed with paraformaldehyde sliced thinly, and stained with hematoxylin eosin (HE). Observation results of the cell structure through the HE staining are shown in Fig. 1. It was confirmed that the produced cell structure includes a connective tissue structure (a of Fig. 1) and an epithelial structure (b of Fig. 1) placed on the connective tissue structure. No tissue shrinkage and tissue cracking were confirmed in the produced cell structure.

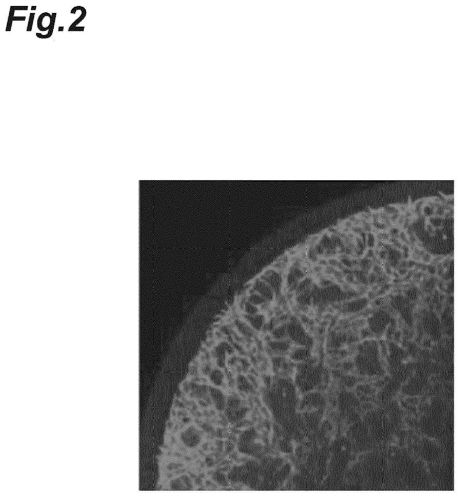

It was confirmed that CMFs were arranged between the cells of the connective tissue structure of the produced cell structure. Formation of a vascular network was confirmed in the connective tissue structure of the produced cell structure through immunostaining using anti-CD31 antibodies (manufactured by Dako) (Fig. 2).

<Test Example 3: Production of Cell Structure and Evaluation of Infiltration>

$2.0 \times 10^7$ cells of *P. gingivalis* were seeded in a cell structure for evaluation obtained in the same manner as in the method described in Test Example 2 except that the cell structure contained no HUVEC (HUVEC was not used when a connective tissue structure was formed). *P. gingivalis* which had been cultured on an agar medium were peeled off from the medium, and then suspended in a mixed medium from which an antibiotic was removed and which was the same as that when an epithelial structure was produced, and seeded on the epithelial structure.

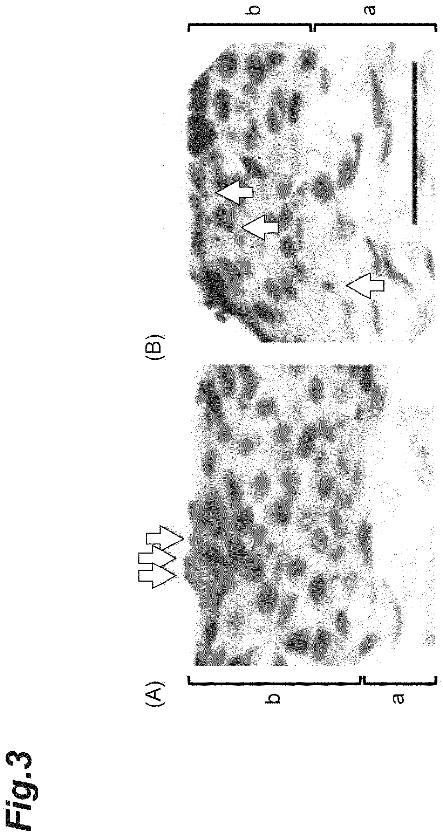

Observation results of the structure for evaluation immediately after the bacteria are seeded and after elapse of 4 hours from the seeding (from infection) are shown in Fig. 3. a of Fig. 3 indicates a connective tissue structure, and b of Fig. 3 indicates an epithelial structure. The arrows in Fig. 3(B) indicate bacteria. The scale bar in Fig. 3 indicates 50 μm.

As shown in Fig. 3, bacteria are moved to the inside of the connective tissue structure and the epithelial structure.

<Test Example 4: Confirmation of Engraftment and Infiltration of *P. gingivalis*>

A cell structure for evaluation obtained in the same manner as in the method described in Test Example 2 except that the cell structure contained no HUVEC (HUVEC was not used when a connective tissue structure was formed) was used. *P. gingivalis* were seeded in the cell structure for evaluation in the same manner as that described in Test Example 3 except that the seeding amount was set to $2.4 \times 10^7$ cells.

The cell structures 24 hours and 48 hours after seeding *P. gingivalis* were peeled off from the insert, and DNA extraction and real-time PCR were carried out. The DNA extraction was carried out using R2081 manufactured by Zymo Research through the method as described in its manual. The real-time PCR was carried out using a kit (THUNDERBIRDR Probe qPCR RT Set (manufactured by TOYOBO) through the method as described in its manual.

Two types of *P. gingivalis* were used: a wild type and a mutant type (Δkpg rgpA rgpB) lacking a protease. The results are shown in Table 1. "N.D." in Table 1 indicates no detection.

Since gingival tissues in a living body have barrier properties due to strong adhesion between cells, it is known that it is necessary for proteins that contribute to adhesion between cells to be decomposed by proteases in order for periodontal disease bacteria (*P. gingivalis*) to be infiltrated into the gingival tissues. According to the results of this test, the number of bacteria infiltrated was significantly reduced in a case where periodontal disease bacteria lacking a protease are used, as shown in Table 1. From this, it was shown that expression of proteases is necessary for periodontal disease bacteria to be well infiltrated into a cell structure produced using CMFs and that the cell structure produced using CMFs is a tissue model closer to a living body.

[Table 1]

| Sample | Incubator time | | STDEV (24 h) | STDEV (48 h) |
|---|---|---|---|---|
| | 24 h | 48 h | | |
| Control (without *P. gingivalis*) | N.D. | N.D. | - | - |
| Wild type (ATCC 33277) | 46787140 | 40875716 | 9206623 | 14159581 |
| Mutant type (△kpg rgpA rgpB) | 13623747 | 5101111 | 3996801 | 166698 |

<Test Example 5: Confirmation (qRT-PCR) of Expression States of Adhesion Factors>

A cell structure for evaluation obtained in the same manner as in the method described in Test Example 2 except that the cell structure contained no HUVEC (HUVEC was not used when a connective tissue structure was formed) was used as a sample with CMFs.

A cell structure for evaluation obtained in the same manner as in the method described in Test Example 2 except that no CMFs were used and the cell structure contained no HUVEC (HUVEC was not used when a connective tissue structure was formed) was used as a sample without CMFs.

A cell structure obtained by culturing Epi4 1 day after seeding it was peeled off from the insert, and mRNA extraction and real-time PCR were carried out. Extraction of mRNA was carried out using PureLink™ RNA Mini Kit manufactured by Invitrogen through the method as described in its manual. The real-time PCR was carried out using a kit (SYBRR Green Realtime PCR Master Mix (manufactured by TOYOBO) through the method as described in its manual.

Confirmation results of expression states of adhesion factors are shown in Table 2. As shown in Table 2, in a case where CMFs were used, cell structures became gingival models closer to a living body due to a high amount of adhesion factors expressed compared to a case where no CMFs were used. $0^{*1}$ indicates 0.00004596, $0^{*2}$ indicates 0.000008649, and $0^{*3}$ indicates 0.0000406.

[Table 2]

| | Absolute value (with CMFs) | Absolute value (without CMFs) | STDEV (with CMFs) | STDEV (without CMFs) |
|---|---|---|---|---|
| Claudin -1 | 1 | 0.059 | 0.449 | 0.025 |
| ZO-1 | 1 | $0^{*1}$ | 0.082 | $0^{*3}$ |
| E-cadherin | 1 | 0.04 | 0.212 | 0.005 |
| Occludin-1 | 1 | 0.001 | 0.257 | 0.002 |
| Collagen IV | 1 | $0^{*2}$ | 0.027 | 0 |
| Laminin | 1 | 0.135 | 0.36 | 0.051 |

<Test Example 6: Production of Cell Structure Having Vascular Network>

1. Preparation of Type I Collagen Solution (10 mg/mL)

50 mg of freeze-dried porcine skin-derived type I collagen (provided by NH Foods Ltd.) was fractionated into a centrifuge tube, 5 mL of 10× PBS (pH 7.4) was added thereto, and the mixture was homogenized for 6 minutes. Centrifugation was performed at 10000 rpm for 3 minutes, pipetting and voltex were performed, and centrifugation was performed again at 10000 rpm for 3 minutes. After sucking a supernatant, 5 mL of DMEM (containing no serum) was added, a precipitate was pipetted firmly, and centrifugation was performed at 10000 rpm for 3 minutes. Thereafter, a supernatant was removed by suction, and 8% FBS-containing DMEM was added so that the content of type I collagen homogenized was 10 mg/mL. After suspension, a required amount was fractionated into an Eppendorf tube. The average length of the obtained defibrated collagen (CMFs) was 22.5 μm, and the average diameter thereof was about 4.4 μm.

2. Construction of Connective Tissue Structure (Connective Tissue Layer) Having Vascular Network

HGF (1.0 × 10^6 cells/tissue) and HUVEC (5.0 × 10^5 cells/tissue) were mixed with a type I collagen solution containing type I collagen at a concentration of 10 mg/mL, and the mixture was seeded in an insert. 0.5 mL of a mixed medium of 8% FBS-DMEM and EGM-2 (Lonza) (1:1) was added to the outside of the insert, centrifugation was performed at 1100 × g for 15 minutes, and the mixture was cultured at 37°C. The next day, the 24-well insert was transferred to a 6-well plate and cultured in 12 mL of a mixed medium of 8% FBS-containing DMEM and EGM-2 (1:1) for 1 week.

3. Construction of Epithelial Structure (Epithelial Cell Layer)

The medium inside the insert of the connective tissue structure was sucked, PBS (type IV collagen solution) containing type IV collagen at a concentration of 0.04 mg/mL was added to the inside of the insert, and the mixture was incubated at 37°C for 20 minutes or longer. Meanwhile, a cell suspension of IHGE cells was prepared using a mixture of 8% FBS-containing DMEM: EGM-2:2%FBS-containing HuMedia-KG2 (Kurabo Industries Ltd.)=1:1:1. After sucking the type IV collagen solution inside the insert and the medium outside the insert, epithelial cells were seeded inside the insert, the mixed medium was added to the outside of the insert, and they were allowed to stand for 37°C for 1 hour. Thereafter, the mixed medium was further added to the outside of the insert, and the inside and the outside of the insert were connected to each other in the case of the 24-well insert to perform culture overnight. Accordingly, a gingival model tissue consisting of a cell structure in which an epithelial structure was placed on a connective tissue structure was constructed.

4. Culture of *P. gingivalis*

*P. gingivalis* ATCC 33277 was inoculated into a blood agar medium (BD) and cultured at 37°C while maintaining an anaerobic condition. Proliferation culture to a logarithmic proliferation period was performed using a trypticase soybroth (TBS) medium (BD) to which heamin (5 $\mu$g/mL, Sigma-Aldrich) and menadione (1 $\mu$g/mL, Sigma-Aldrich) were added.

5. Infection with *P. gingivalis*

*P. gingivalis* was adjusted with a mixed medium of antibiotic-free 8% FBS-containing DMEM:EGM-2:2% FBS-containing HuMedia-KG2 (Kurabo Industries Ltd.)=1:1:1 so as to become 2.4 × 10^7 cells/tissue, and was administered to a gingival model tissue. After administration, *P. gingivalis* was cultured in a 5% $CO_2$ incubator at 37°C.

6. Immunohistochemical Analysis with *P. gingivalis*

A section of a *P. gingivalis*-infected gingival model tissue was incubated overnight with Anti-*P. Gingivalis* Antibody Produced in Rabbit (Sigma-Aldrich) at 4°C. Thereafter, the section was treated with Horseradish Peroxidase Conjugated Goat Anti-Rabbit IgG (Nichirei Corporation) and was then subjected to color development with a 3,3'-diaminobenzidine substrate solution (DAKO). Counterstaining was performed with haematoxylin.

7. *P. Gingivalis* Invasion Assay

*P. gingivalis* was stained with a -Bacstain-CTC Rapid Staining Kit (Dojindo Laboratories). The *P. gingivalis* was suspended in PBS, and the suspension was incubated with 5-cyano-2,3-ditolyl tetrazolium chloride (CTC) and an enhancing reagent B at 37°C for 30 minutes. After washing a gingival model tissue with PBS, the stained *P. gingivalis* was administered to the gingival model tissue and co-cultured in a 5% $CO_2$ incubator at 37°C.

*P. gingivalis* was labeled with a fluorescent dye before administration to the gingival model tissue. -Bacstain-CTC Rapid Staining Kit (for Microscopy) was used for labeling. This kit detects NAD(P)H generated in cells due to electron transfer representing a respiratory activity. CTC is reduced due to this NAD(P)H to become fluorescent formazan ($\lambda$ex=about 430,480 nm, $\lambda$em=620 to 640 nm). Since this fluorescent formazan is deposited in cells and observed, this can be observed in individual cells. Since an enhancer contained in the kit promotes a reduction reaction of CTC due to NAD(P)H, high-sensitivity detection is possible.

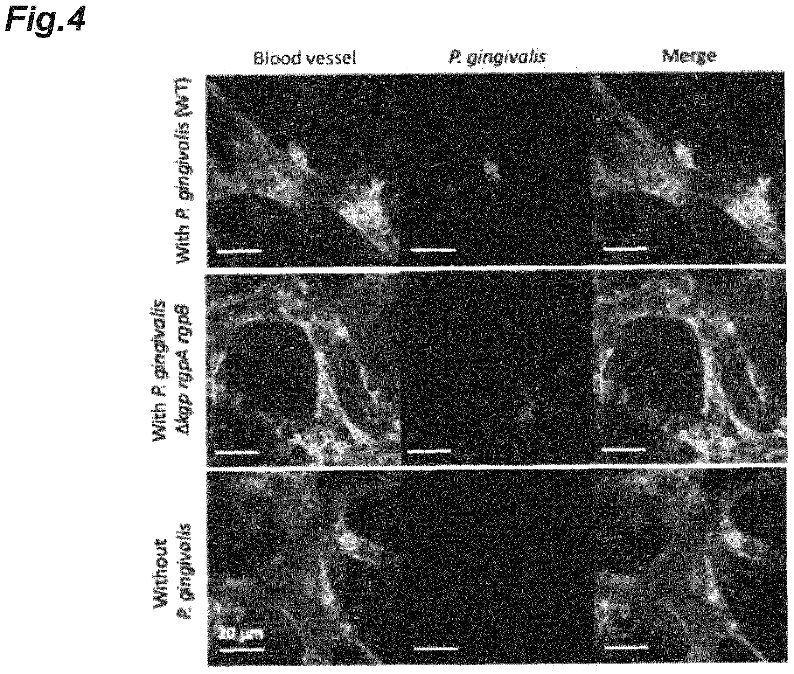

Fig. 4 shows an image of *P. gingivalis* labeled with an anti-CD31 antibody and CTC. Fig. 4 shows that *P. gingivalis* is colocalized with vascular network structures. It became clear from the photograph shown in Fig. 4 that *P. gingivalis* could invade vascular network structures of gingival model tissues. A signal showing colocalization was observed also in the gingival model tissue infected with a cell strain (*P. gingivalis* Δkgp ΔrgpA ΔrgpB) lacking gingipains which are enzymes having a trypsin-like activity, but was lower than that of the model infected with *P. gingivalis* WT. The above-described results suggest that the gingipains likely to be involved in movement of *P. gingivalis* to a deep portion of a gingival tissue and infiltration of capillaries.

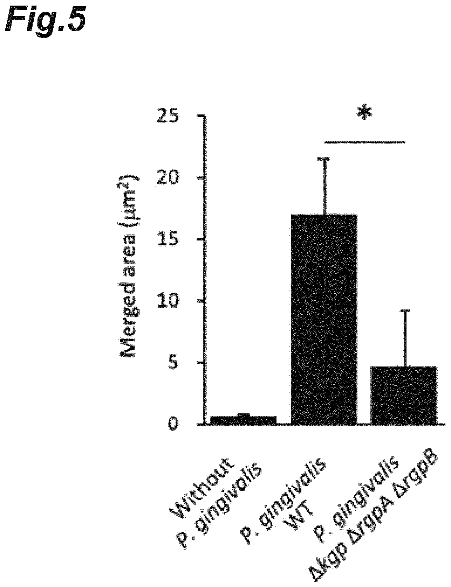

In addition to the image shown in Fig. 4, two similar images were acquired (a total of three sheets), area values of regions where *P. gingivalis* was colocalized with a vascular network structure were analyzed with Image J to calculate an average value. The calculation results are shown in Fig. 5. As shown in Fig. 5, it was confirmed that *P. gingivalis* was infiltrated into a vascular network similarly to the results confirmed from the image of Fig. 4. Infiltration of *P. gingivalis* into a vascular network was more significant in WT than in the gingipain-deficient strain. In Fig. 5, * indicates that there is a significant difference ($p < 0.05$).

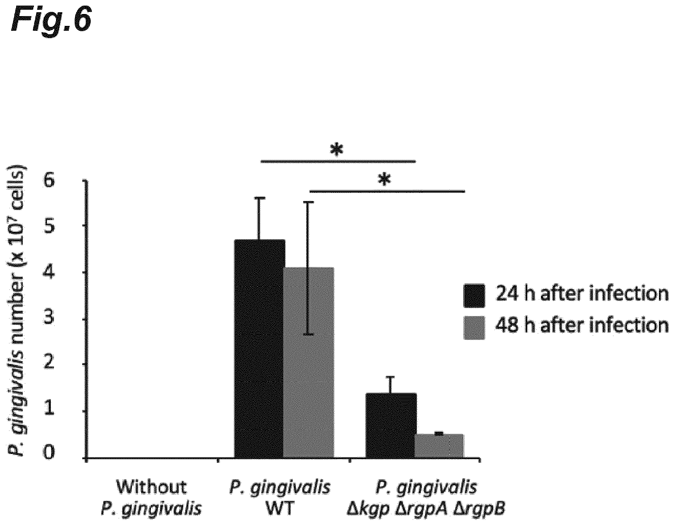

Fig. 6 shows quantitative evaluation results of the number of *P. gingivalis* infiltrated into each gingival model tissue. The number of *P. gingivalis* in gingival model tissues after "5. Infection with *P. gingivalis*" of Test Example 6 was measured through real-time PCR. The real-time PCR was carried out through the same method as that in Test Example 4. * indicates that there is a significant difference ($p < 0.05$).

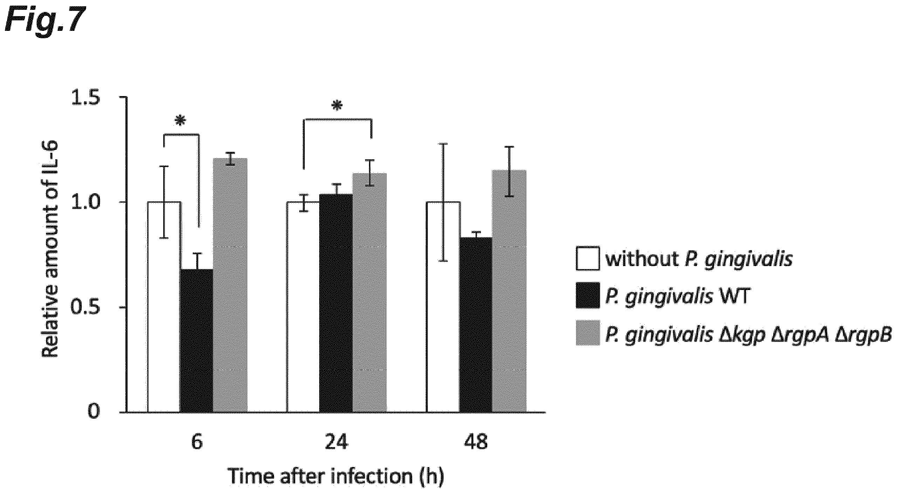

Fig. 7 shows evaluation results of the amount of IL-6 secreted after *P. gingivalis* infection. The amounts of IL-6 secreted from gingival model tissues after "5. Infection with *P. gingivalis*" of Test Example 6 were measured and compared with each other. IL-6 is a cytokine generally used as an indicator of inflammation. In general, an increase in the amount of IL-6 indicates that inflammation is occurring, that is, infection is occurring.

According to the graph shown in Fig. 7, the one in which WT was seeded had a low amount of IL-6 compared to the gingipain-deficient strain and one in which *P. gingivalis* was not seeded. This is considered that gingipains have an action of decomposing IL-6. The amount of IL-6 was increased in the gingipain-deficient strain compared to one in which *P. gingivalis* was not seeded. This indicates that infection is occurring when the data shown in Fig. 4 and the quantitative evaluation results of the number of *P. gingivalis* shown in Fig. 6 are considered together.

IL-6 was quantitatively determined through ELISA. Culture supernatants were collected 6 hours, 24 hours, and 48 hours after each gingival model tissue was infected with *P. gingivalis,* and the amount of IL-6 in each culture supernatant was measured manually using ELISA Max kits (BioLegend). In Fig. 7, * indicates that there is a significant difference ($p < 0.05$).

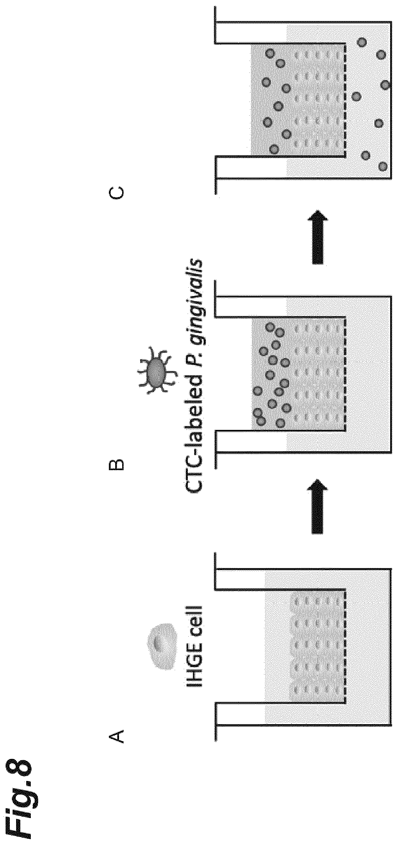

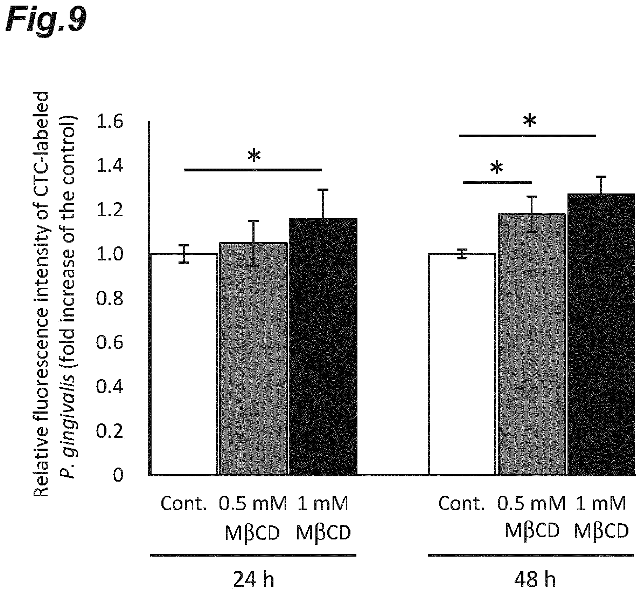

Figs. 8 and 9 are views illustrating an observation method and observation results of behavior of *P. gingivalis* permeating an epithelial cell layer.

In a living body, it is necessary for *P. gingivalis* to penetrate an epithelial cell layer to move from the surface of a gingival tissue to a connective tissue layer. Some bacteria are known to interact with lipid rafts to promote invasion into host cells. According to the following experiment, it was confirmed that lipid rafts may be involved in permeation of *P. gingivalis* into an epithelial cell layer and that *P. gingivalis* may pass through cells, not between cells as a primary route for movement to a deeper region of a gingival tissue.

Fig. 8 shows a method for observing behavior of *P. gingivalis* permeating an epithelial cell layer. In this method, methyl-β-cyclodextrin (MβCD) which was a substance capable of reversibly removing cholesterol from the plasma membrane of cells was used.

First, $1.0 \times 10^6$ IHGE cells were seeded in a 24-well plate Transwell (manufactured by Corning Incorporated) having a void volume of 3 μm and cultured in a Humedia-KG2 medium for 1 day to produce an epithelial structure (epithelial cell layer tissue) (A of Fig. 8) consisting of only an epithelial cell layer.

Next, MβCD was dissolved in dimethyl sulfoxide (DMSO) to obtain an MβCD solution having a final concentration of MβCD of 50 mM or 100 mM. Subsequently, the above-described MβCD solution was mixed with a Humedia-KG2 medium (the final concentration of MβCD in the medium: 0.5 mM or 1.0 mM). After the epithelial cell layer tissue was washed with PBS, the above-described Humedia-KG2 medium containing MβCD was added to the epithelial cell layer tissue in Transwell to incubate the epithelial cell layer tissue in the Humedia-KG2 medium at 37°C for 30 minutes.

Subsequently, CTC-labeled *P. gingivalis* was prepared in the same procedure as above, and the epithelial cell layer tissue was infected with the CTC-labeled *P. gingivalis* (B of Fig. 8). After the infection, incubation was performed in the Humedia-KG2 medium containing MβCD at 37°C, and the amounts of CTC-labeled *P. gingivalis* which had penetrated into the epithelial cell layer tissue from the upper portion of Transwell and permeated the lower portion of the 24-well plate 24 hours or 48 hours after the infection were analyzed with a spectrofluorometer FP-8500 (manufactured by JASCO) (C of Fig. 8). The analysis results are shown in Fig. 9.

In the structure in which the epithelial cell layer tissue was treated with MβCD, the amount of *P. gingivalis* permeated was significantly increased compared to the structure in which the epithelial cell layer tissue was not treated therewith. It was confirmed that this effect was increased in a concentration-dependent manner. These results show that lipid rafts are involved in migration of *P. gingivalis* to an epithelial cell layer. In addition, it is suggested that an intracellular route may be used instead of an intercellular route as a primary route for *P. gingivalis* to move to a deep portion of a gingival tissue.

**Claims**

1. A cell structure comprising:

   a connective tissue structure; and
   an epithelial structure placed on the connective tissue structure,
   wherein the connective tissue structure comprises a fragmented extracellular matrix component and first cells comprising mesenchymal cells,
   wherein at least a part of the fragmented extracellular matrix component is placed between the first cells, and
   wherein the epithelial structure comprises epithelial cells.

2. The cell structure according to claim 1,
   wherein the mesenchymal cells comprise fibroblasts.

3. The cell structure according to claim 1 or 2,
   wherein the fragmented extracellular matrix component comprises a fragmented collagen component.

4. The cell structure according to any one of claims 1 to 3,

   wherein the first cells further comprise vascular endothelial cells, and
   wherein the connective tissue structure has a vascular network between the first cells.

5. The cell structure according to claim 4,
   wherein the connective tissue structure has a vascular network between the mesenchymal cells.

6. A bacteria-containing tissue model comprising:

   the cell structure according to any one of claims 1 to 5; and
   bacteria arranged in the cell structure or arranged so as to come into contact with the epithelial structure of the cell structure.

7. The bacteria-containing tissue model according to claim 6,
   wherein the bacteria are periodontal disease bacteria.

8. A method for producing a cell structure, the method comprising:

   a first step of bringing a fragmented extracellular matrix component into contact with first cells comprising mesenchymal cells and culturing the first cells to form a connective tissue structure; and
   a second step of bringing the connective tissue structure into contact with second cells comprising epithelial cells and culturing the connective tissue structure and the second cells to form an epithelial structure on the connective tissue structure and obtain a cell structure.

9. The method for producing a cell structure according to claim 8, wherein the first cells comprise vascular endothelial cells.

10. The method for producing a cell structure according to claim 8 or 9,
    wherein the amount of the fragmented extracellular matrix component when the fragmented extracellular matrix component is brought into contact with the first cells is 0.1 to 100 mg per $1.0 \times 10^6$ cells.

11. A method for producing a bacteria-containing tissue model, the method comprising:
    a step of bringing bacteria into contact with the epithelial structure of the cell structure obtained through the production method according to any one of claims 8 to 10.

12. The method for producing a bacteria-containing tissue model according to claim 11, the method further comprising:
    a step of culturing the cell structure and the bacteria after the step of bringing the bacteria into contact with the epithelial structure to move the bacteria to the inside of the cell structure.

13. A method for evaluating behavior of a cell structure and/or bacteria, the method comprising:

a step of evaluating behavior of the cell structure and/or the bacteria after bringing the bacteria into contact with the epithelial structure of the cell structure according to any one of claims 1 to 5.

## Fig.1

(A)

1mm

(B)

b

a

200 μm

EP 3 991 797 A1

*Fig.2*

Fig.3

Fig.4

# *Fig.5*

## Fig.6

Fig.7

# *Fig.8*

A

IHGE cell

B

CTC-labeled *P. gingivalis*

C

Fig.9

## INTERNATIONAL SEARCH REPORT

| International application No. |
| --- |
| PCT/JP2020/032446 |

### A. CLASSIFICATION OF SUBJECT MATTER

A61P 43/00(2006.01)i; A61P 17/00(2006.01)i; C12N 5/071(2010.01)i; C12N 1/20(2006.01)i; C12Q 1/02(2006.01)i; A61K 35/12(2015.01)i; A61K 35/33(2015.01)i; A61K 35/35(2015.01)i; A61K 35/36(2015.01)i; A61K 35/44(2015.01)i; A61L 27/24(2006.01)i; A61L 27/36(2006.01)i; A61L 27/38(2006.01)i; A61L 27/40(2006.01)i; A61L 27/60(2006.01)i

FI: C12N5/071; C12N1/20 A; C12Q1/02; A61L27/24; A61L27/36 130; A61L27/38 200; A61L27/40; A61K35/36; A61L27/60; A61P17/00; A61K35/44; A61K35/33; A61K35/12; A61K35/35; A61P43/00 121

According to International Patent Classification (IPC) or to both national classification and IPC

### B. FIELDS SEARCHED

Minimum documentation searched (classification system followed by classification symbols)

A61P43/00; A61P17/00; C12N5/071; C12N1/20; C12Q1/02; A61K35/12; A61K35/33; A61K35/35; A61K35/36; A61K35/44; A61L27/24; A61L27/36; A61L27/38; A61L27/40; A61L27/60

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2018/143286 A1 (TOPPAN PRINTING CO., LTD.) 09 August 2018 (2018-08-09) claims, paragraphs [0014], [0032], [0050], examples 3, 10 | 1–5, 8–10 |
| Y | | 6, 7, 11–13 |
| X | WO 2019/093477 A1 (TOPPAN PRINTING CO., LTD.) 16 May 2019 (2019-05-16) claims, example 3 | 1–3, 8, 10 |
| Y | | 6, 7, 11–13 |
| X | JP 2010-172247 A (THE KITASATO INSTITUTE) 12 August 2010 (2010-08-12) paragraphs [0017], [0038], example 1 | 1–3, 8, 10 |
| Y | | 6, 7, 11–13 |

☒ Further documents are listed in the continuation of Box C.  ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 October 2020 (20.10.2020) | 02 November 2020 (02.11.2020) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2020/032446 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 2008-508968 A (PHENION GMBH & CO. KG) 27 March 2008 (2008-03-27) claims 9, 10, paragraphs [0062], [0063] | 6, 7, 11-13 |
| Y | CN 109971704 A (INST OF DERMATOLOGY AND HOSPITAL FOR SKIN DESEASES CHINESE ACADEMY OF MEDICAL SCIENCES) 05 July 2019 (2019-07-05) example 2 | 6, 7, 11-13 |
| Y | JP 2010-502175 A (BASF BEAUTY CARE SOLUTIONS FRANCE SAS) 28 January 2010 (2010-01-28) claims 22, 24 | 6, 7, 11-13 |
| A | JP 2018-83794 A (KOSÉ CORPORATION) 31 May 2018 (2018-05-31) paragraph [0002] | 1-13 |
| A | US 2004/0013712 A1 (PARMA, Bruna) 22 January 2004 (2004-01-22) entire text | 1-13 |

Form PCT/ISA/210 (continuation of second sheet) (January 2015)

<table>
<tr><td colspan="3" align="center">**INTERNATIONAL SEARCH REPORT**<br>Information on patent family members</td><td>International application No.<br><br>PCT/JP2020/032446</td></tr>
</table>

| Patent Documents referred in the Report | Publication Date | Patent Family | Publication Date |
|---|---|---|---|
| WO 2018/143286 A1 | 09 Aug. 2018 | US 2019/0351098 A1 paragraphs [0032], [0057], [0077], examples 3, 10 EP 3578638 A1 CN 110050059 A | |
| WO 2019/093477 A1 | 16 May 2019 | (Family: none) | |
| JP 2010-172247 A | 12 Aug. 2010 | US 2011/0281351 A1 paragraphs [0041], [0083], example 1 | |
| JP 2008-508968 A | 27 Mar. 2008 | US 2007/0269792 A1 claims 11, 12, paragraphs [0070], [0071] WO 2006/018147 A2 EP 1778307 A2 CN 101001652 A | |
| CN 109971704 A | 05 Jul. 2019 | (Family: none) | |
| JP 2010-502175 A | 28 Jan. 2010 | US 2010/0297765 A1 paragraphs [0096]-[0099] WO 2008/028882 A1 EP 2064318 A1 KR 10-2009-0049620 A | |
| JP 2018-83794 A | 31 May 2018 | (Family: none) | |
| US 2004/0013712 A1 | 22 Jan. 2004 | WO 2002/009790 A1 EP 1307247 A1 | |

Form PCT/ISA/210 (patent family annex) (January 2015)

**EP 3 991 797 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015072164 A
- WO 2017146124 A
- WO 2018143286 A

**Non-patent literature cited in the description**

- *Acta Biomaterialia,* 2015, vol. 25, 131-142